# EUROPEAN PATENT SPECIFICATION

(11) **EP 3 471 826 B1**
(45) Date of publication and mention of the grant of the patent: **30.06.2021**
(21) Application number: 17812720.5
(22) Date of filing: 14.06.2017
(51) Int. Cl.: A61B 18/20, A61N 5/06

(54) **DEVICES AND METHODS TO REMOVE HAIR**
VORRICHTUNGEN UND VERFAHREN ZUR ENTFERNUNG VON HAAR
DISPOSITIFS ET PROCÉDÉS POUR ENLEVER DES POILS

(30) Priority: 15.06.2016 US 201662350193 P
(43) Date of publication of application: 24.04.2019
(73) Proprietor: Hong Kong Health and Beauty Limited, Hong Kong (CN)
(72) Inventor: CHEN, Haiying, Hong Kong (CN)
(74) Representative: Cabinet Chaillot
(86) International application number: PCT/CN2017/088260
(87) International publication number: WO 2017/215609

(56) References cited:
- WO-A1-2014/076503
- CN-A- 101 232 853
- CN-A- 101 938 951
- CN-A- 101 977 560
- CN-A- 104 721 968
- CN-U- 204 563 343
- US-A1- 2003 233 138
- US-A1- 2005 177 141
- US-A1- 2007 167 999
- US-A1- 2007 208 395
- US-A1- 2007 270 785
- US-A1- 2008 027 518
- US-A1- 2010 211 136
- US-A1- 2012 010 603
- US-A1- 2012 116 373
- US-A1- 2012 165 682
- US-A1- 2012 253 331
- US-A1- 2016 129 279

## Description

### FIELD OF INVENTION

The present invention relates to devices and methods useful in hair removal. The apparatuses can, for instance, permanently remove unwanted human or animal hair.

### BACKGROUND OF INVENTION

In order to remove unwanted hair, some electronic devices use light, such as intense pulsed light (IPL) or laser light. Electronic devices using IPL to remove hair are often bulky and require a large circuit board and fan. Electronic devices using laser light to remove hair can pose hazards to the user. For example, continuous irradiation can lead to skin redness, blistering, burns, and other side effects. Additionally, laser light emits a monochromatic light that is not suitable for different skin types.

US20120165682A1 discloses a hair removal device utilizes a system for sensing the presence and color of skin by means of skin color sensor assembly and one or more light-emitting diodes as defined in the preamble of claims 1 respectively.

US20070270785A1 discloses a light-based treatment method and apparatus for removing unwanted hair from a target region of the skin by means of multiple wavelength bands of light or radiation according to a skin type as defined in the preamble of claims 1 respectively.

WO2014076503A1 discloses a handhold device for light treatment of skin by means of an array of light-emitting diodes divided into different groups and selection circuitry as defined in the preamble of claims 1 respectively.

US20120116373A1 discloses a light application apparatus for applying light to an object by means of a light source and a light detector as defined in the preamble of claims 1 respectively.

US20120253331A1 discloses a dermatological treatment device for delivering the generated laser beam to the target area of tissue by means of a laser diode bar and power source.

US20050177141A1 discloses a system and method for dermatological treatment, such as hair removal by means of a power supply controlling the amount of current density through a lamp.

US20080027518A1 discloses a dermatologic hair-regrowth-inhibiting apparatus for hair-regrowth-inhibiting by means of a light source to produce output light pulses.

US20070208395A1 discloses a method and apparatus for treating a target body surface using a radiation applicator by means of a light source.

US20160129279A1 discloses a wearable device for therapeutic irradiation of skin comparing a light source for light-emitting, a proximity sensor for detecting proximity of light spreading sheet to the skin.

US20120010603A1 disclose a tissue optical clearing devices for subsurface photodisruption and methods of use generally comprise an energy source in conjunction with mechanical optical clearing for the creation of high precision surface and subsurface photodisruption and/or photoablation.

US20070270785A1 discloses a light-based treatment method and apparatus removed unwanted hair from a target region of skin. The treatment method used multiple wavelength bands of light or radiation. The ratio of the energies of the wavelength bands is selected according to a skin parameter, e.g., skin type, which can be differentiated by the amount of melanin in the skin.

New methods and apparatus for removing unwanted hair are desired.

### SUMMARY OF INVENTION

One example embodiment is an electronic device to remove unwanted hair on a target area. The electronic device includes a LED unit that includes at least two LEDs that emit light with different wavelengths from each other; a skin type detector including a light source and a photoreceptor, that detects a skin type of a target area; and a LED driver that electrically communicates with the skin type detector and transmits power to the LED unit through a plurality of separate output channels. Peak wavelengths of the LEDs range from 600nm - 1200nm, and each of the two LEDs in the LED unit is independently powered by one of the separate output channels; the skin type detector, upon receiving a signal from the LED driver, detects the skin type by illuminating the light source on the skin surface of the target area, and reading its reflection light with the photoreceptor, according to the reflected light, the skin type detector generates a signal of the skin type to the LED driver, based on the received signal, the LED driver powers suitable type of LED in the LED unit for following hair removing process to achieve better results and avoid side effects, the skin types are classified using Fitzpatrick skin classifications, and the LEDs emitting light with wavelengths of 694nm, 790nm, 808nm, 810nm, 850nm/945nm or 1064nm is applied to skin type I, II, III, IV, V and VI, respectively, the two LEDs are powered with a current pulse of 1A - 5A, a pulse width of 20ms - 400ms, and an off time period of 0.5 - 10 second (s).

Other example embodiments are discussed herein.

### BRIEF DESCRIPTION OF FIGURES

Fig. 1 shows an electronic device for hair removal in accordance with an example embodiment.
Fig. 2 shows a LED unit including three different types of LEDs (L1, L2, and L3) that emit light with different peak wavelengths in accordance with an example embodiment.
Fig. 3a, Fig. 3b, and Fig. 3c show spot shapes of an effective irradiation area in accordance with example embodiments.
Fig. 4a and 4b show comparisons of overlap areas between square spot shapes with circular spot shapes of the effective irradiation area in accordance with example embodiments.
Fig. 5 shows a schematic diagram of a flexibly wearable part including LED unit in accordance with an example embodiment.
Fig. 6 shows a hair removal process in accordance with an example embodiment.
Fig. 7 shows a table with suggested LED wavelength to be used for different skin types in accordance with an example embodiment.
Fig. 8 shows a test result of an output energy density and an effective irradiation area size of the skin with an LED in accordance with an example embodiment.
Fig. 9a, Fig. 9B, Fig. 9c, and Fig. 9d show a schematic diagram of circuit layouts in accordance with example embodiments.

### DETAILED DESCRIPTION OF THE PREFERRED EMBODIMENTS

As used herein and in the claims, "comprising" means including the following elements but not excluding others.

As used herein and in the claims, "connect" refers to electrical coupling or connection either directly or indirectly via one or more electrical means unless otherwise stated.

An example embodiment includes an electronic device and methods that removes unwanted hair from a person or animal utilizing light emitting diodes (LEDs) as the light source.

LED spectrum has a narrow Full width at half maximum (FWHM) in the range of tens of nanometers. The peak wavelength can be changed by adjusting a band gap energy of the materials forming the p-n junction. LED is low voltage device, and the drive circuit is safe. Pulse interval thereof can also be short, greatly reducing the time of charging so as reducing the waiting time for users. Customer experience is improved.

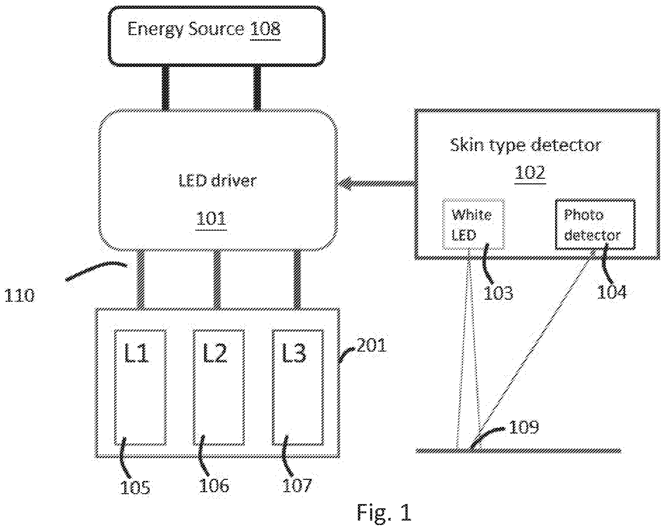

An example embodiment is an electronic hair removal device that includes a LED driver 101, a LED unit 201, and a skin type detector 102, as shown in Fig. 1.

The LED driver 101 electrically connects with the LED unit 201, the skin type detector 102, and an energy or power source 108. The LED driver 101 communicates with the skin type detector 102 to send and receive signals, and further powers the LED unit 201 via a plurality of separate output channels 110.

The LED unit 201 comprises two or more types of LEDs that emit light with different wavelengths or frequencies from the electromagnetic spectrum. For example, L1, L2, and L3 each emit light with a different frequency or wavelength from each other.

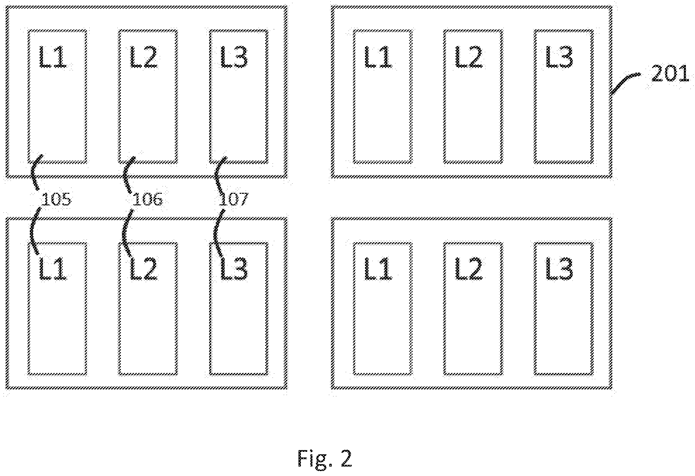

Fig. 2 shows four LED units, one LED unit incorporates three types of LEDs, L1 105, L2 106, and L3 107. Peak wavelengths of the LEDs, according to an example embodiment, range from 600 nanometers (nm) to 1200nm. In a further example embodiment, the peak wavelength is 694nm, 790nm, 808nm, 810nm, or 1064nm. The spectrums of L1 105, L2 106 and L3 107 are different with each other. In a further example embodiment, L1's spectrum peaks at 690nm, while L2 at 810nm and L3 at 1064nm. In another example embodiment, the peak wavelength and numbers of LED could be changed as required.

Each LED L1, L2 or L3 in the LED unit 201 is independently powered by one of the separate output channels. Every output channel is independently controlled and modulated by the LED driver 101 to achieve different combination of light. In one example, two or more LEDs emit light at same portion of the target area.

The skin type detector 102 comprises a light source 103 and a photoreceptor 104 to detect a skin type of a target area 109. In a further example embodiment, the light source is a white light LED.

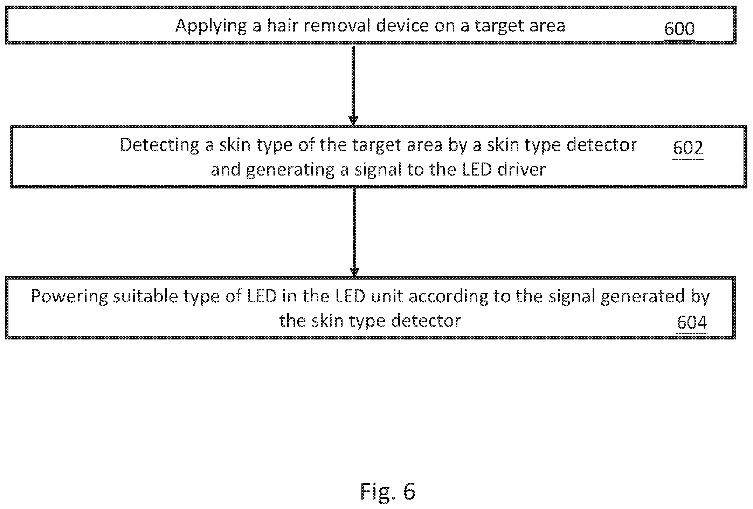

In one example embodiment, a method of removing unwanted human or animal hair using the hair removal device described above is provided as shown in Fig. 6.

Block 600 states applying a hair removal device on a target area 109. The skin type detector 102, upon receiving a signal from the LED driver 101, detects the skin type by illuminating the light source 103 on the skin surface of the target area, and reading its reflection light with the photoreceptor 104 as stated in Block 602. According to the reflected light, the skin type detector 102 generates a signal of the skin type to the LED driver 101. Block 604 states that the LED driver 101, based on the received signal, powers suitable type of LED in the LED unit 201 for following hair removing process to achieve better results and avoid side effects.

The skin types are classified using the Fitzpatrick skin classifications, consisting of type I (pale white), type II (white), type III (cream white), type IV (light brown), type V (brown) and type VI (dark brown). In a further example embodiment, if fairer skin type is detected or determined, LEDs with short wavelength will be turn on and others locked. For darker skin type, only LEDs with longer wavelength will be turned on and others locked. Turning on and off the LEDs in this manner reduces or eliminates redness or even blisters of the skin that are present with conventional electronic hair removal devices.

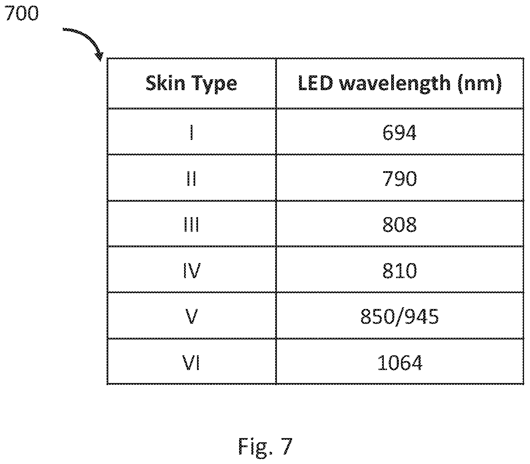

A table 700 in Fig. 7 shows suggested LED wavelength to be used for different skin types. The LEDs emitting light with wavelengths of 694nm, 790nm, 808nm, 810nm, 850nm/945nm or 1064nm is applied to skin type I, II, III, IV, V and VI, respectively.

In another example embodiment, the LED L1, L2 or L3 is powered with a current pulse of 1 - 5 Amps (A), a pulse width of 20 - 400 milliseconds (ms) and an off time of 0.5 - 10 second (s). In another example, the LED L1, L2 or L3 is powered with a current pulse of 2A, a pulse width of 390 ms, and an off time of 2s.

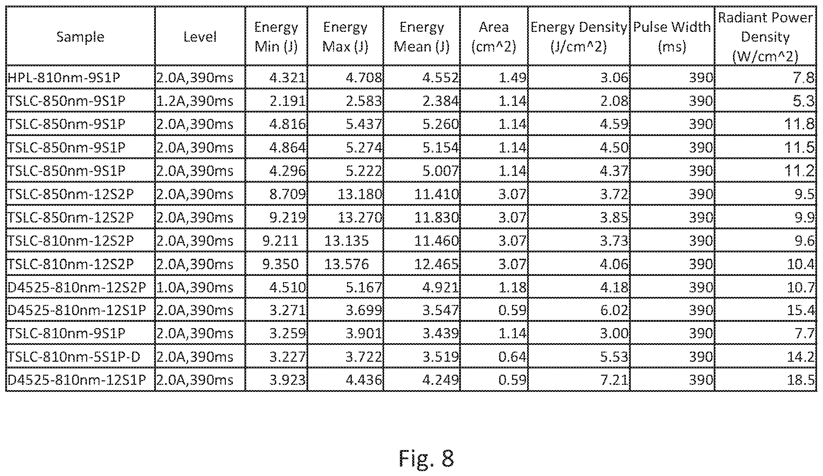

Fig. 8 shows a test result of an output energy density and an effective irradiation area size of the skin with an LED light in accordance with an example embodiment. The results show that the output density and the area size are sufficient for home use hair removal device.

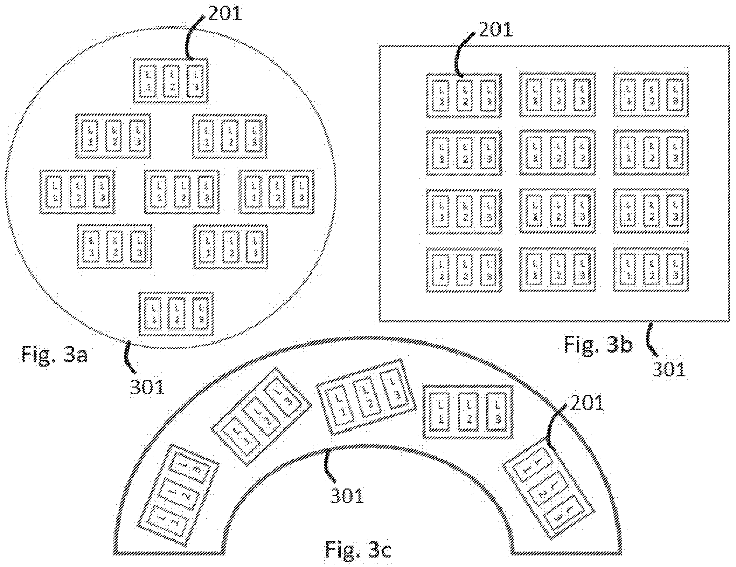

In another example embodiment, a spot of an effective irradiation area 301 of the hair removal device is circular, square, or irregular, in order to achieve a particular location irradiation and hair removal. Fig. 3a, Fig. 3b and Fig. 3c show the spot shape of plural LEDs on a printed circuit board 302 (not shown).

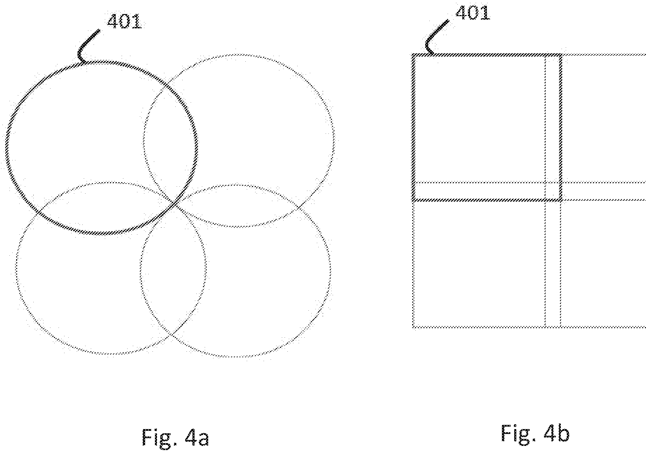

In another example embodiment, LED unit further comprises a lens that can achieve a square or rectangular beam spot shape 401. By this way, an overlap area may be reduced when applying light on a large skin area. This enables an example embodiment to provide quicker treatment process and reducing side effects when compared with conventional electronic hair removal devices. Fig. 4 shows a comparison of overlap areas between square spot shapes with circular spot shapes.

In another example embodiment, LED L1, L2 and L3 are small and are placed on a flexible circuit board. A surface of the LED unit is encapsulated with a flexible material to achieve a deformable light source such that it can be applied on uneven arears of the skin while achieving uniform hair removal results. In a further example embodiment, the flexible material is silicone.

In another example embodiment, LED unit 201 and the skin detector 102 are located adjacent to the LED driver 101.

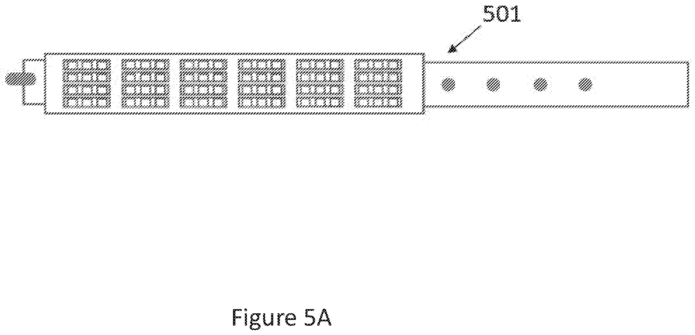

In another example embodiment, LED unit 201 and the skin detector 102 are separated from the LED driver 101 but in electrical communication with it. LED unit is placed on a flexible circuit board and encapsulated with a flexible material such that the LED unit is configured as a flexibly wearable part 501. In a further example embodiment, the flexibly wearable part 501 is in a wristband shape, and can be apply on limbs or other body parts of a person easily and quickly, as shown in Fig. 5.

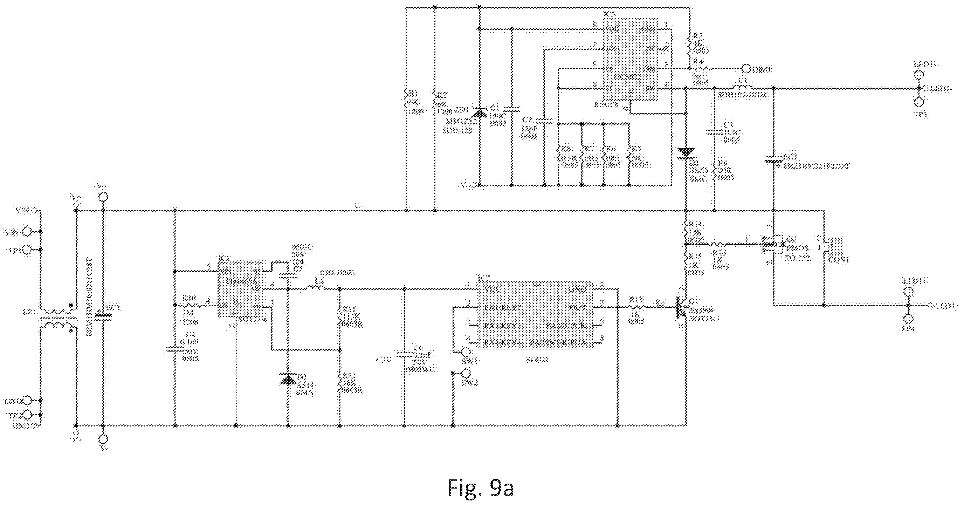

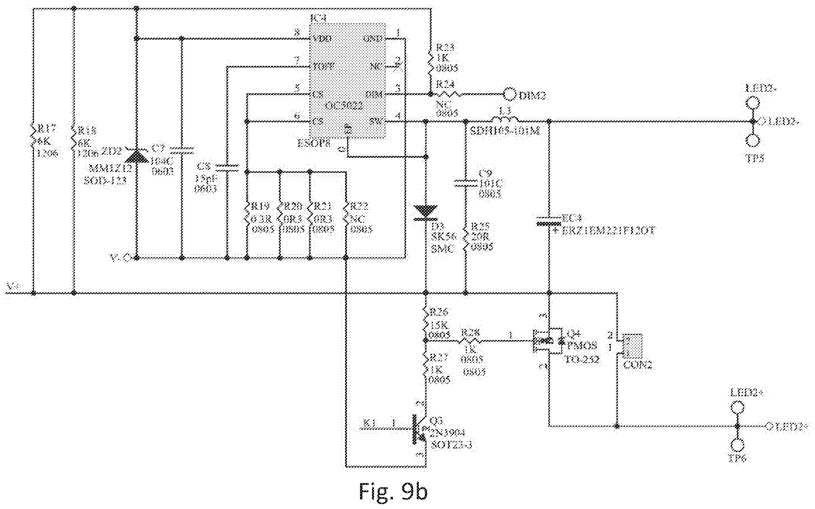

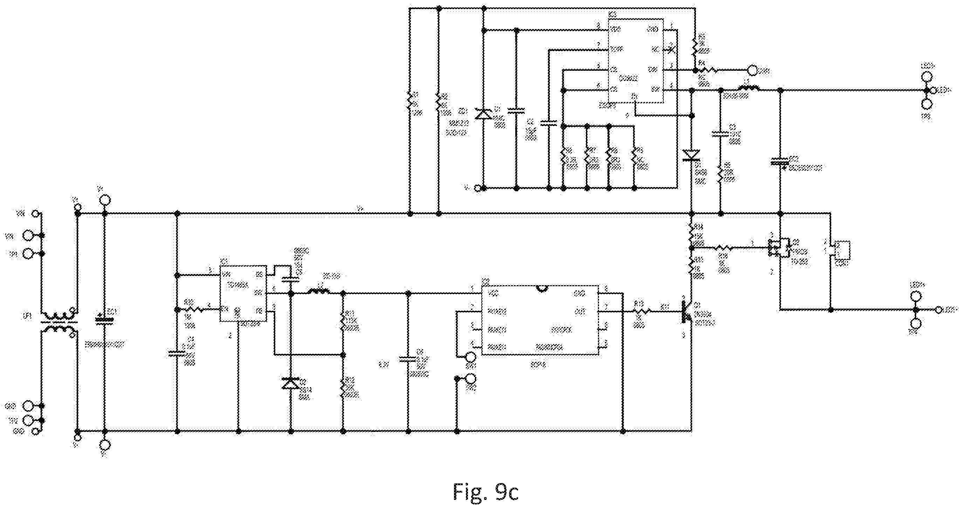

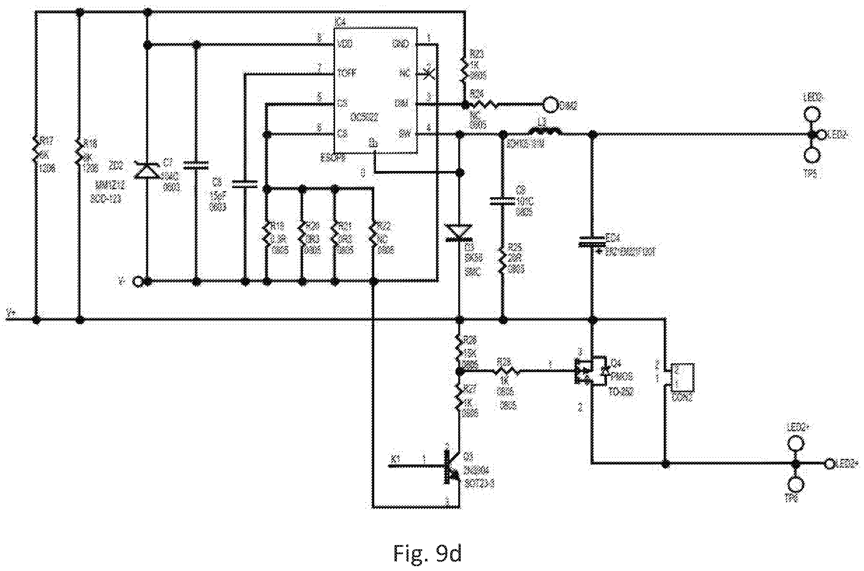

In one example embodiment, a circuit of a constant voltage chip is shown in Fig. 9a to convert an input voltage of 24V into an output voltage of 3.3V for a circuit of controller chip of Fig. 9b. In another example embodiment, the circuit of Fig. 9b modulates constant current chips to power the LED L1, L2 or L3 with a current pulse of 1 - 5 Amps (A), a pulse width of 20 - 400 milliseconds (ms) and an off time of 0.5 - 10 second (s). Circuits of constant current chips are shown in Fig. 9c and Fig. 9d.

The exemplary embodiments of the present invention are thus fully described. Although the description referred to particular embodiments, it will be clear to one skilled in the art that the present invention may be practiced with variation of these specific details. Hence this invention should not be construed as limited to the embodiments set forth herein.

## Claims

1. An electronic device to remove unwanted hair on a target area, comprising:
a LED unit (201) that includes at least two LEDs that emit light with different wavelengths from each other
wherein the LED unit (201) is configured to emit light with wavelengths of 694nm, 790nm, 808nm, 810nm, 850nm/945nm or 1064nm.
a skin type detector (102) including a light source (103) and a photoreceptor (104), that detects a skin type of a target area; and
a LED driver (101) that electrically communicates with the skin type detector (102) and transmits power to the LED unit (201) through a plurality of separate output channels (110),
wherein peak wavelengths of the LEDs range from 600nm - 1200nm, and each of the two LEDs in the LED unit (201) is independently powered by one of the separate output channels(110), wherein
the skin type detector (102), upon receiving a signal from the LED driver (101), detects the skin type by illuminating the light source (103) on the skin surface of the target area, and reading its reflection light with the photoreceptor (104), according to the reflected light, the skin type detector (102) generates a signal of the skin type to the LED driver (101), based on the received signal, the LED driver (101) powers suitable type of LED in the LED unit for following hair removing process to achieve better results and avoid side effects,
the electronic is configured to classify the skin types using Fitzpatrick skin classifications, and to apply the LEDs emitting light with wavelengths of 694nm, 790nm, 808nm, 810nm, 850nm/945nm or 1064nm to skin type I, II, III, IV, V and VI, respectively and
the two LEDs are powered with a current pulse of 1A - 5A, a pulse width of 20ms - 400ms, and an off time period of 0.5 - 10 second (s).

2. The electronic device according to claim 1, wherein the LED unit (201) includes three types of LEDs that each emit light with different wavelengths from each other.

3. The electronic device according to claim 1, wherein the LED unit (201) includes three LEDs, and each of the three LEDs emit a different wavelength that corresponds to a different skin type.

4. The electronic device according to claim 1, wherein a spot of an effective irradiation area of the device is circular, square, or irregular.

5. The electronic device according to claim 1, wherein the LED unit (201) is encapsulated with a flexible material to make the LED unit (201) deformable.

6. The electronic device according to claim 1, wherein the LED unit is flexible and wearable on a person.

7. The electronic device according to claim 1, wherein the LED unit is shaped as a wearable wristband.

8. A cosmetic method of removing hair using the electronic device of claim 1, comprising:
applying light emitted from the electronic device on a target area;
detecting, with the skin type detector (102) upon receiving a signal from the LED driver (101), a skin type of the target area by illuminating the light source (103) on the skin surface of the target area, and reading its reflection light with the photoreceptor (104);
generating, by the skin type detector (102) according to the reflected light, with the electronic device and based on the skin type detected, a signal to the LED driver (101); and
powering, by the LED driver (101), based on the received signal, LEDs in the LED unit (201) according to the signal generated by the skin type detector (102) for following hair removing process to achieve better results and avoid side effects;
the skin types are classified using Fitzpatrick skin classifications, and the LEDs emitting light with wavelengths of 694nm, 790nm, 808nm, 810nm, 850nm/945nm or 1064nm is applied to skin type I, II, III, IV, V and VI, respectively, the two LEDs are powered with a current pulse of 1A - 5A, a pulse width of 20ms - 400ms, and an off time period of 0.5 - 10 second (s)

## Patentansprüche

1. Elektronische Vorrichtung zur Entfernung von unerwünschtem Haar auf einem Zielbereich, umfassend:
eine LED-Einheit (201), die mindestens zwei LEDs beinhaltet, die Licht mit voneinander verschiedenen Wellenlängen emittieren, wobei die LED-Einheit (201) konfiguriert ist, um Licht mit Wellenlängen von 694 nm, 790 nm, 808 nm, 810 nm, 850 nm/945 nm oder 1064 nm zu emittieren;
einen Hauttyp-Detektor (102), darin eingeschlossen eine Lichtquelle (103) und einen Fotorezeptor (104), der einen Hauttyp eines Zielbereichs detektiert; und
einen LED-Treiber (101), der elektrisch mit dem Hauttyp-Detektor (102) kommuniziert und Strom an die LED-Einheit (201) durch eine Vielzahl von getrennten Ausgabekanälen (110) überträgt,
wobei Spitzen-Wellenlängen der LEDs im Bereich von 600 nm - 1200 nm liegen und jede der zwei LEDs in der LED-Einheit (201) unabhängig von einem der getrennten Ausgabekanäle (110) mit Strom versorgt wird, wobei der Hauttyp-Detektor (102) beim Empfang eines Signals vom LED-Treiber (101) den Hauttyp durch Beleuchtung der Lichtquelle (103) auf der Hautfläche des Zielbereichs detektiert und beim Ablesen ihres Reflexionslichts mit dem Fotorezeptor (104) gemäß dem reflektierten Licht der Hauttyp-Detektor (102) ein Signal des Hauttyps an den LED-Treiber (101) basierend auf dem empfangenen Signal erzeugt, der LED-Treiber (101) eine geeignete Art von LED in der LED-Einheit mit Strom für den folgenden Haarentfernungsprozess versorgt, um bessere Ergebnisse zu erzielen und Nebenwirkungen zu vermeiden,
die Elektronik konfiguriert ist, um die Hauttypen unter Verwendung von Fitzpatrick-Hautklassifizierungen zu klassifizieren, und um die LEDs anzuwenden, die Licht mit Wellenlängen von 694 nm, 790 nm, 808 nm, 810 nm, 850 nm/945 nm oder 1064 nm auf den Hauttyp I, II, III, IV, V bzw. VI emittieren und die zwei LEDs mit einem Stromimpuls von 1A - 5A, einer Impulsbreite von 20 ms - 400 ms und einer Auszeit von 0,5 - 10 Sekunden versorgt werden.

2. Elektronische Vorrichtung nach Anspruch 1, wobei die LED-Einheit (201) drei Arten von LEDs beinhaltet, die jeweils Licht mit voneinander verschiedenen Wellenlängen emittieren.

3. Elektronische Vorrichtung nach Anspruch 1, wobei die LED-Einheit (201) drei LEDs beinhaltet und jede der drei LEDs eine verschiedene Wellenlänge emittiert, die einem verschiedenen Hauttyp entspricht.

4. Elektronische Vorrichtung nach Anspruch 1, wobei ein Punkt eines effektiven Bestrahlungsbereichs der Vorrichtung rund, quadratisch oder unregelmäßig ist.

5. Elektronische Vorrichtung nach Anspruch 1, wobei die LED-Einheit (201) mit einem flexiblen Material gekapselt ist, um die LED-Einheit (201) verformbar zu machen.

6. Elektronische Vorrichtung nach Anspruch 1, wobei die LED-Einheit flexibel und an einer Person tragbar ist.

7. Elektronische Vorrichtung nach Anspruch 1, wobei die LED-Einheit als ein tragbares Armband geformt ist.

8. Kosmetisches Verfahren zum Entfernen von Haar unter Verwendung der elektronischen Vorrichtung nach Anspruch 1, umfassend:
Anwenden von Licht, emittiert von der elektronischen Vorrichtung, auf einen Zielbereich;
Detektieren, mit dem Hauttyp-Detektor (102), beim Empfang eines Signals vom LED-Treiber (101), eines Hauttyps des Zielbereichs durch Beleuchten der Lichtquelle (103) auf der Hautfläche des Zielbereichs und Ablesen ihres Reflexionslichts mit dem Fotorezeptor (104);
Erzeugen, durch den Hauttyp-Detektor (102) gemäß dem reflektierten Licht, mit der elektronischen Vorrichtung und basierend auf dem detektierten Hauttyp, eines Signals an den LED-Treiber (101); und
Versorgen mit Strom, durch den LED-Treiber (101), basierend auf dem empfangenen Signal, von LEDs in der LED-Einheit (201) gemäß dem Signal, das durch den Hauttyp-Detektor (102) erzeugt wird, für den folgenden Haarentfernungsprozess, um bessere Ergebnisse zu erzielen und Nebenwirkungen zu vermeiden;
die Hauttypen werden unter Verwendung von Fitzpatrick-Hautklassifizierungen klassifiziert, und die LEDs, die Licht mit Wellenlängen von 694 nm, 790 nm, 808 nm, 810 nm, 850 nm/945 nm oder 1064 nm emittieren, werden auf den Hauttyp I, II, III, IV, V bzw. VI angewendet, die zwei LEDs werden mit einem Stromimpuls von 1A - 5A, einer Impulsbreite von 20 ms - 400 ms und einer Auszeit von 0,5 - 10 Sekunden mit Strom versorgt.

## Revendications

1. Dispositif électronique pour éliminer des poils indésirables sur une zone cible, comprenant :
une unité DEL (201) qui comprend au moins deux DEL qui émettent de la lumière avec des longueurs d'onde différentes l'une de l'autre, l'unité DEL (201) étant configurée pour émettre de la lumière avec des longueurs d'onde de 694 nm, 790 nm, 808 nm, 810 nm, 850 nm/945 nm ou 1064 nm,
un détecteur de type de peau (102) comprenant une source de lumière (103) et un photorécepteur (104), qui détecte un type de peau d'une zone cible ; et
un contrôleur de DEL (101) qui communique électriquement avec le détecteur de type de peau (102) et transmet une alimentation à l'unité DEL (201) par une pluralité de canaux de sortie séparés (110),
des longueurs d'onde de crête des DEL variant entre 600 nm et 1200 nm, et chacune des deux DEL dans l'unité DEL (201) étant indépendamment alimentée par l'un des canaux de sortie séparés (110),
le détecteur de type de peau (102), lors d'une réception d'un signal provenant du contrôleur de DEL (101), détectant le type de peau par éclairage de la source de lumière (103) sur la surface de peau de la zone cible, et lecture de sa lumière de réflexion avec le photorécepteur (104), en fonction de la lumière réfléchie, le détecteur de type de peau (102) générant un signal du type de peau vers le contrôleur de DEL (101), sur la base du signal reçu, le contrôleur de DEL (101) alimentant un type approprié de DEL dans l'unité DEL pour suivre un processus d'élimination de poils pour obtenir de meilleurs résultats et éviter des effets secondaires, l'électronique étant configurée pour classer les types de peau à l'aide de classifications de peau de Fitzpatrick, et pour appliquer les DEL émettant de la lumière avec des longueurs d'onde de 694 nm, 790 nm, 808 nm, 810 nm, 850 nm/945 nm ou 1064 nm à un type de peau I, II, III, IV, V et VI, respectivement et les deux DEL étant alimentées avec une impulsion de courant de 1 A à 5 A, une largeur d'impulsion de 20 ms à 400 ms, et une période de temps de coupure de 0,5 à 10 secondes.

2. Dispositif électronique selon la revendication 1, dans lequel l'unité DEL (201) comprend trois types de DEL qui émettent chacune de la lumière avec des longueurs d'onde différentes les unes des autres.

3. Dispositif électronique selon la revendication 1, dans lequel l'unité DEL (201) comprend trois DEL, et chacune des trois DEL émet une longueur d'onde différente qui correspond à un type de peau différent.

4. Dispositif électronique selon la revendication 1, dans lequel un spot d'une zone d'irradiation efficace du dispositif est circulaire, carré, ou irrégulière.

5. Dispositif électronique selon la revendication 1, dans lequel l'unité DEL (201) est encapsulée avec un matériau flexible pour rendre l'unité DEL (201) déformable.

6. Dispositif électronique selon la revendication 1, dans lequel l'unité DEL est flexible et peut être portée sur une personne.

7. Dispositif électronique selon la revendication 1, dans lequel l'unité DEL est façonnée sous la forme d'un bracelet pouvant être porté.

8. Procédé cosmétique d'élimination de poils utilisant le dispositif électronique selon la revendication 1, comprenant :
appliquer de la lumière émise par le dispositif électronique sur une zone cible ;
détecter, avec le détecteur de type de peau (102) lors d'une réception d'un signal provenant du contrôleur de DEL (101), un type de peau de la zone cible par éclairage de la source de lumière (103) sur la surface de peau de la zone cible, et lecture de sa lumière de réflexion avec le photorécepteur (104) ;
générer, par le détecteur de type de peau (102) en fonction de la lumière réfléchie, avec le dispositif électronique et sur la base du type de peau détecté, un signal vers le contrôleur de DEL (101) ; et
alimenter, par le contrôleur de DEL (101), sur la base du signal reçu, des DEL dans l'unité DEL (201) en fonction du signal généré par le détecteur de type de peau (102) pour suivre un processus d'élimination de poils pour obtenir de meilleurs résultats et éviter des effets secondaires ;
les types de peau étant classés à l'aide de classifications de peau de Fitzpatick, et les DEL émettant de la lumière avec des longueurs d'onde de 694 nm, 790 nm, 808 nm, 810 nm, 850 nm/945 nm ou 1064 nm étant appliquées à un type de peau I, II, III, IV, V et VI, respectivement, les deux DEL étant alimentées avec une impulsion de courant de 1 A à 5 A, une largeur d'impulsion de 20 ms à 400 ms, et une période de temps de coupure de 0,5 à 10 secondes.
